# EUROPEAN PATENT APPLICATION

(11) **EP 2 543 381 A1**
(43) Date of publication of application: **09.01.2013**
(21) Application number: 11750388.8
(22) Date of filing: 03.03.2011
(51) Int. Cl.: A61K 36/00, A23L 1/30, A61P 11/00, A61P 31/04, A61P 31/12, A61P 37/08, A61P 43/00

(54) **IMMUNOGLOBULIN A SECRETION PROMOTER**

(30) Priority: 04.03.2010 JP 2010047689
(71) Applicant: Lotte Co., Ltd., Tokyo 160-0023 (JP)
(72) Inventor: ISHIZUKA, Tomokazu, Saitama-shi Saitama 336-8601 (JP); KIYONO, Hiroshi, Tokyo 113-0033 (JP); KUNISAWA, Jun, Tokyo 113-0033 (JP); YOSHIDA, Keishiro, Saitama-shi Saitama 336-8601 (JP); SHIMURA, Susumu, Saitama-shi Saitama 336-8601 (JP)
(74) Representative: Blodig, Wolfgang
(86) International application number: PCT/JP2011/001261
(87) International publication number: WO 2011/108275

(57) **Abstract**

Most of conventionally known IgA antibody secretion promoters relate to IgA antibody production in the intestine, and there are known few IgA antibody secretion promoters relating to an IgA secretion promoting action and an infection suppressing action in organs of the respiratory system such as the oral cavity, the nasal cavity, and the respiratory tract. Thus, provided is a pharmaceutical composition having an IgA secretion promoting action in organs of the respiratory system such as the oral cavity and the respiratory tract as well as in the intestine. An IgA secretion promoter containing a corn poppy extract has an IgA secretion promoting action in the oral cavity and the respiratory tract as well as an IgA secretion promoting effect in the intestine. Therefore, ingestion of the extract can suppress infection with viruses and bacteria and prevent onset of allergy.

## Description

### Technical Field

The present invention relates to an IgA secretion promoter which can be expected to exhibit an effect of preventing infection with bacteria, viruses, and the like and an effect of preventing allergy, and more particularly, to a composition which can be expected to exhibit an effect of promoting IgA secretion in the upper respiratory tract.

### Background Art

In surfaces located inside of a living body and in contact with the outside, such as the oral cavity, the respiratory tract, the gastrointestinal tract, and the like, a strong defense system called mucosal immunization is established in order to prevent invasion of viruses, bacteria, and the like from the outside to the living body. On a mucous membrane surface, mucus is constantly secreted from the saliva gland, the mucous gland, and the like, which physically prevents invasion of viruses and bacteria and defends the living body against foreign invaders by virtue of actions of an antibody, an antimicrobial peptide, an enzyme, and the like present in the mucus. An immunoglobulin A (IgA) antibody plays a central role in this defense system, having actions of, for example, removing viruses, bacteria, and the like and preventing an adherence thereof to the mucous membrane surface (Non Patent Literature 1).

Concerning a relationship between the IgA antibody and the infection, there is a report that an increase in IgA antibody which is specific for a pathogen of otitis media in an animal experiment suppressed infection with the pathogen (Non Patent Literature 2). Further, there is a report that administration of an IgA antibody which is specific for a certain kind of pathogenic virus of a cold to the human nasal cavity suppressed infection with the virus (Non Patent Literature 3).

Further, it is considered that the IgA antibody not only has a role in defending the living body against infection with bacteria, viruses, and the like, but also it blocks a foreign antigen which causes an allergic reaction from entering through the mucous membrane to the living body (Non Patent Literature 4).

Under the above-mentioned background, it is estimated that promotion of secretion of the IgA antibody should be effective for preventing infection with bacteria, viruses, and the like and for preventing allergy.

Hitherto, various lactic acid bacteria and basidiomycetes (Patent Literatures 1 to 4), and polysaccharides and oligosaccharides (Patent Literatures 5 to 9 and Non Patent Literature 5) have been disclosed as IgA antibody secretion promoters having an IgA antibody secretion promoting action. However, most of the IgA antibody secretion promoters relate to IgA antibody production in the intestine, and there are known few IgA antibody secretion promoters relating to an IgA secretion promoting action and an infection suppressing action in organs of the respiratory system such as the oral cavity, the nasal cavity, and the respiratory tract (Patent Literature 2 and Patent Literature 10).

### Citation List

### Patent Literature

PTL 1: Japanese Patent No. 2968374
PTL 2: Japanese Patent No. 3818319
PTL 3: Japanese Patent Application Laid-Open No. H11-92389
PTL 4: Japanese Patent Application Laid-Open No. 2005-97133
PTL 5: Japanese Patent No. 4162147
PTL 6: Japanese Patent Application Laid-Open No. 2001-64181
PTL 7: Japanese Patent Application Laid-Open No. 2003-201239
PTL 8: Japanese Patent Application Laid-Open No. 2006-70217
PTL 9: Japanese Patent Application Laid-Open No. 2006-213671
PTL 10: Japanese Patent Application Laid-Open No. 2009-161447
PTL 11: Japanese Patent Application Laid-Open No. 2002-370993

### Non Patent Literature

NPL 1: Brandtzaeg P., International Journal of Medical Microbiology, 2003, vol. 293, No. 1, pages 3-15, "Role of secretory antibodies in the defence against infections."
NPL 2: Hotomi M. et al., Vaccine, 1998, vol. 16, No. 20, pages 1950-1956, "Specific mucosal immunity and enhanced nasopharyngeal clearance of nontypeable Haemophilus influenza after intranasal immunization with outermembrane protein P6 and cholera toxin."
NPL 3: Heikkinen T. et al., Pediatric Infectious Disease Journal, 1998, vol. 17, No. 5, pages 367-372, "Intranasally administered immunoglobulin for the prevention of rhinitis in children."
NPL 4: Zenro Ikezawa, "Development of low allergenic food" CMC Publishing CO., LTD, pages 41-49.
NPL 5: Scholtens P.A. et. al., Journal of Nutrition, 2008, vol. 138, No. 6, pages 1141-1147, "Fecal secretory immunoglobulin A is increased in healthy infants who receive a formula with short-chain galacto-oligosaccharides and long-chain fructo-oligosaccharides."

### Summary of Invention

### Technical Problem

In view of the above-mentioned problem, an object of the present invention is to provide a pharmaceutical composition having an IgA secretion promoting action in the oral cavity and the respiratory tract as well as in the intestine.

### Solution to Problem

The inventors of the present invention have made extensive studies. As a result, the inventors have found that a corn poppy extract has an IgA secretion promoting action. Thus, the present invention has been completed.

### Advantageous Effects of Invention

The corn poppy extract of the present invention has an IgA secretion promoting action in the oral cavity and the respiratory tract as well as an IgA secretion promoting effect in the intestine. Therefore, the ingestion of the extract can suppress infection with viruses and bacteria and prevent the onset of allergy.

### Description of Embodiments

Corn poppy (scientific name: *Papaver rhoeas L.*), which has been found to have an IgA secretion promoting action in the present invention, is a papaveraceous plant also called field poppy. The plant is said to have, for example, sedative, hypnotic, and analgesic actions, and thus it is used as an antitussive drug or a sedative agent.

The patent literature relating to an immunostimulant containing a herb (Patent Literature 11) discloses poppy as an example of the herb. However, the literature lacks the specificity of the invention and does not have any description on IgA.

The corn poppy extract to be used in the present invention may be obtained by extracting flowers, leaves, and stems of corn poppy, preferably flowers of corn poppy, through the use of water or a mixture of water with a hydrophilic organic solvent.

No particular limitation is imposed on a method for the extraction and the kind of the solvent. As the solvent, there may be used, in addition to water, alcohols such as ethanol, methanol, propanol, glycerin, and propylene glycol, organic solvents such as an ether, acetone, ethyl acetate, chloroform, and hexane, and a combination thereof. In this regard, however, the extraction is desirably carried out through the use of water, ethanol, or a mixed solution thereof from the viewpoint of safety.

Conditions for the extraction for obtaining an extract are preferably 40 to 90°C and about 0.5 to 5 hours, though the extraction may be carried out at any temperature of high temperature, room temperature, and low temperature.

The extract may be used in a form of a solution. Powder obtained by further filtering the solution and distilling off an extraction solvent, followed by drying under reduced pressure or lyophilization, or a concentrate of the solution may also be used. Further, a fractionation purification product of the extract by organic solvent fraction, column chromatography, or the like may be used. A purification treatment such as deodorization or decolorization may be additionally carried out to the extract, in addition.

The content of the corn poppy extract is not particularly limited. It is 0.01% by weight or more, preferably 0.1 to 90% by weight in terms of dry weight with respect to a food and beverage or a preparation.

An ingestion method for the corn poppy extract is not particularly limited. Any known method is applicable such as oral ingestion, ingestion by sublingual administration, ingestion by transnasal administration, or direct local administration such as subcutaneous or intravascular administration.

A pharmaceutical composition containing the corn poppy extract of the present invention may be utilized as, for example, a drug, a quasi drug, or a food and beverage.

In the case of the drug, the pharmaceutical composition is administered as an oral preparation. A dosage form of the oral preparation is exemplified by a pill, a capsule, a powder, a syrup, and a drinkable preparation. In the case of the quasi drug, the pharmaceutical composition is utilized in dosage forms such as a pill, a capsule, and a drinkable preparation. In the case of the food and beverage, there are given, for example, beverages such as a drink, foods such as a candy, a rice cracker, and a cookie, favorite articles such as tobacco and chewing gum, a feed or feeds, and cosmetics such as a gargle and a dentifrice. It should be noted that they are merely illustrative, and the present invention may be used in any article.

In the case of the drug or the quasi drug, one kind or two or more kinds of pharmacologically acceptable carriers, and active ingredients for other treatments as necessary, may be contained. In addition, an excipient, a binder, a disintegrant, a lubricant, a dispersant, a surfactant, a plasticizer, a suspension, an emulsifier, a diluent, a buffer, an antioxidant, a bacterial inhibitor, and the like may be contained.

Further, the food and beverage may contain a substrate, a carrier, an excipient, an additive, an extender, a colorant, a flavoring agent, and the like that are acceptable in food manufacture.

It should be noted that ingredients except the corn poppy extract are also not limited to the foregoing, and any known ingredient may be optionally contained in the present invention.

The drug, quasi drug, or food and beverage containing the corn poppy extract of the present invention may be manufactured by any method known in each technical field. The corn poppy extract may be added by any known method in a manufacturing process therefor.

It should be noted that the pharmaceutical composition containing the corn poppy extract of the present invention may be used for not only humans but also animals except humans (hereinafter, abbreviated as non-human animals). Examples of the non-human animals may include animals except humans, such as mammals, reptiles, amphibians, and fish.

### Examples

Hereinafter, the present invention is described by way of examples. It should be noted that the present invention is by no means limited to these examples.

### Example 1

### Preparation of hot water extract

100 g of corn poppy flowers were pulverized. Water was then added in a 10-fold amount with respect to the weight of the sample, and then extraction was carried out at 70°C for 2 hours. Insoluble matter was filtered off and the extract was then lyophilized to give 37.5 g of a hot water extract.

### Example 2

In vitro evaluation of IgA antibody production promoting action using Peyer's patch-derived lymphocyte cells
The small intestine of mice (BALB/c, female, 6-week-old) was extirpated, and the Peyer's patches were harvested and immersed in an RPMI 1620 medium containing 2% fetal calf serum (FCS). The Peyer's patches were cut with scissors, and lymphocyte cells were collected with a cell strainer (manufactured by BD Biosciences, pore diameter: 70 µm). The collected cell suspension was centrifuged, and the supernatant was removed by suction. A complete medium was then added to adjust the cell concentration of the lymphocyte cell suspension to 4×10⁶ cells/ml. An RPMI 1620 medium containing 10% FCS, 50 µM 2-mercaptoethanol, 1 mM sodium pyruvate, 100 U/ml penicillin, and 100 µg/ml streptomycin was used as the complete medium. 0.05 ml of a culture medium having dissolved therein the lymphocyte cell suspension and the sample (the concentration of the sample was adjusted so that the final concentration was 100 ppm) was added to each of wells of a 96-well plate and cultured under the conditions of 37°C and 5% CO₂ for 1 week. The culture liquid was collected and centrifuged. The total amount of an IgA antibody in the supernatant was then measured by a sandwich ELISA method. The total amount of the IgA antibody in a control in which culture was carried out without the addition of the sample was defined as 1, and the total amount of the IgA antibody in the case where culture was carried out with the addition of the sample was calculated as a relative value.

### Measurement of total amount of IgA antibody by sandwich ELISA method

The measurement by the sandwich ELISA method was carried out according to the following procedures.

100 µl of a 1 µg/ml solution of an anti-mouse immunoglobulin antibody were charged into each of wells of a 96-well ELISA plate, and the antibody was adsorbed onto the plate through a reaction at 4°C overnight. The solution was removed from the plate, and 180 µl of phosphate buffered saline (PBS) containing 1% bovine serum albumin (BSA) were charged into each of the wells, followed by incubation at room temperature for 1 hour to block the non-specific adsorption of an undesired protein. Each of the wells was washed with PBS containing 0.05% Tween 20 (PBST), and a sample solution was then added, followed by incubation at room temperature for 2 hours. Each of the wells was washed with PBST, and 25 ng of peroxidase-bound anti-mouse IgA antibody were added to each of the wells, followed by incubation at room temperature for 1 hour. A peroxidase substrate was then added, and the mixture was subjected to a reaction for 2 minutes. 0.5 N hydrochloric acid was added to terminate the reaction, and an absorbance at 450 nm was measured. A calibration curve was prepared through the use of a mouse IgA antibody as a standard substance, and the amount of an IgA antibody was measured.

The results are as shown in Table 1. The amount of the IgA antibody in the medium in the case where the corn poppy (flower) hot water extract was added to the medium at a concentration of 100 ppm was 2.6 times as large as the case without the addition.

**[Table 1]**

| Sample | | Amount of IgA antibody (relative value) |
|---|---|---|
| Plant name | Site | |
| Corn poppy | Flower | 2.6 |

### Example 3

### Evaluation-1 of IgA secretion promoting action by repeated mixed feed administration to mice

A test feed including a normal feed (MF, Oriental Yeast Co., Ltd.) mixed with a corn poppy (flower) hot water extract at 5% (w/w) was prepared. Six 7-week-old mice (BALB/c) were divided into a control group and a test group (three mice for each group). The mice in the control group and the test group were allowed to ingest the normal feed and the test feed ad libitum, respectively, and fed for 6 weeks. The feces of the mice were collected every 1 week after the start of the feeding. Each of the feces was suspended in a phosphate buffer so that the concentration was 100 mg/ml and centrifuged. The total amount of an IgA antibody in the supernatant was then measured by a sandwich ELISA method in the same manner as in Example 2.

The results are shown in Table 2 as relative values in the case where the total amount of the IgA antibody on week 0 for each individual is defined as 1. When the average values were compared with each other, the administration group showed a high relative value for the total amount of the IgA antibody in the feces as compared to the control group.

**[Table 2] Measurement results of IgA level**

| Group | Individual number | Total amount of IgA antibody (value relative to that on week 0 for each individual) | | | | | | |
|---|---|---|---|---|---|---|---|---|
| | | Week 0 | Week 1 | Week 2 | Week 3 | Week 4 | Week 5 | Week 6 |
| Control group | 1 | 1.00 | 0.42 | 0.47 | 0.35 | 0.23 | 0.17 | 0.38 |
| | 2 | 1.00 | 1.27 | 1.58 | 0.42 | 0.58 | 0.37 | 1.03 |
| | 3 | 1.00 | 0.86 | 0.53 | 0.26 | 0.23 | 0.25 | 0.53 |
| | Average | 1.00 | 0.85 | 0.86 | 0.34 | 0.34 | 0.26 | 0.64 |
| Administration group | 4 | 1.00 | 2.42 | 2.54 | 1.78 | 0.63 | 1.25 | 2.21 |
| | 5 | 1.00 | 2.46 | 0.94 | 1.68 | 1.31 | 1.28 | 2.79 |
| | 6 | 1.00 | 0.95 | 0.13 | 0.48 | 0.18 | 0.38 | 1.05 |
| | Average | 1.00 | 1.94 | 1.20 | 1.31 | 0.71 | 0.97 | 2.02 |

### Example 4

### Evaluation-2 of IgA secretion promoting action by repeated mixed feed administration to mice

A test feed including a normal feed (MF, Oriental Yeast Co., Ltd.) mixed with a corn poppy (flower) hot water extract at 5% (w/w) was prepared. Twelve 7-week-old mice (BALB/c) were divided into a control group and a test group (six mice for each group). The mice in the control group and the test group were allowed to ingest the normal feed and the test feed ad libitum, respectively, and fed. On week 4, the mice were immunized with 50 µg of phosphorylcholine-keyhole limpet hemocyanin (PC-KLH, an antigen common to bacteria) by transnasal administration. After that, the transnasal immunization was carried out every 1 week, sinus mucus (100 to 200 µl/mouse) and lymphocyte cells in the lamina propria mucosae in the nasal cavity (1×10⁵ to 5×10⁵ cells/mouse) were collected 1 week after the third transnasal immunization, and the total amount of an IgA antibody, the total number of IgA antibody producing cells, a PC-specific IgA antibody titer, and the number of PC-specific IgA antibody producing cells were measured. Further, the feces were collected, suspended in a phosphate buffer so that the concentration was 100 mg/ml, and centrifuged. The total amount of the IgA antibody in the supernatant was then measured. The total amount of the IgA antibody and the PC-specific IgA antibody titer were measured by a sandwich ELISA method, and the number of the PC-specific IgA antibody producing cells was measured by an enzyme-linked immunospot (ELISPOT) method. Since the number of the lymphocyte cells in the lamina propria mucosae in the nasal cavity capable of being harvested from each animal was very small, the number of the IgA antibody producing cells was measured in duplicate by combining the cells from three mice together.

### Measurement of amount of IgA antibody by sandwich ELISA method

The measurement of the total amount of the IgA antibody was carried out by a sandwich ELISA method in the same manner as in Example 2. Further, the measurement of the amount of the PC-specific IgA antibody was carried out by a sandwich ELISA method in the same manner as in Example 2 except that PC-BSA was used in place of the anti-mouse immunoglobulin antibody to be first adsorbed onto the ELISA plate.

### Measurement of number of IgA antibody producing cells by ELISPOT method

The measurement of the number of PC-specific IgA antibody producing cells by the ELISPOT method was carried out according to the following procedures.

100 µl of an anti-mouse immunoglobulin antibody (5 µg/ml PBS) or PC-BSA (5 µg/ml PBS) were added to a commercially available 96-well nitro cellulose membrane plate for ELISPOT, and adsorbed onto the plate through a reaction at 4°C overnight. The antibody solution was removed, and blocking was carried out with a complete medium. After that, an appropriately diluted suspension of lymphocyte cells from the lamina propria mucosae in the nasal cavity was added, followed by incubation under the conditions of 37°C and 5% CO₂ for 4 hours. After washing with PBS, 100 ng of a peroxidase-bound anti-mouse IgA antibody were added, and the mixture was subjected to a reaction at 4°C overnight. After washing with PBS, a chromogenic substrate (3-amino-9-ethylcarbazole) liquid was added, and color development was carried out under the conditions of room temperature and light shielding for 30 minutes. The resultant was washed with water and dried, and then spots as antibody producing cells were counted under a stereoscopic microscope.

### Measurement of total amount of IgA in feces

The feces were collected, suspended in PBS so that the concentration was 100 mg/ml, and centrifuged. The total amount of IgA in the supernatant was then measured by a sandwich ELISA method in the same manner as in Example 2.

The results are as shown in Table 3 and Table 4. The administration group showed a higher value for the total amount of IgA in both the sinus mucus and the feces, and the administration group also showed a larger value for the total number of IgA producing cells in the lamina propria mucosae in the nasal cavity. This suggests that the mixed feed administration of the corn poppy extract promoted IgA secretion in the intestinal tract and the respiratory system.

**[Table 3]**

| Measurement results of IgA level | | | | | | |
|---|---|---|---|---|---|---|
| | Sinus mucus | | | | Feces | |
| | Total amount of IgA (ng/ml) | | PC-specific IgA antibody titer (Reciprocal log2 titer) | | Total amount of IgA (µg/ml) | |
| | Control group | Administration group | Control group | Administration group | Control group | Administration group |
| Measured value for each individual | 210 | 289 | 0 | 1 | 8.5 | 21.9 |
| | 231 | 734 | 4 | 2 | 5.4 | 8.0 |
| | 260 | 297 | 5 | 5 | 3.7 | 8.8 |
| | 300 | 358 | 4 | 4 | 2.3 | 13.1 |
| | 263 | 299 | 4 | 3 | 2.0 | 4.2 |
| | 211 | 454 | 3 | 4 | 2.1 | 5.2 |
| Average value | 246 | 405 | 3.3 | 3.2 | 4.0 | 10.2 |
| Standard error | 14.2 | 70.5 | 0.7 | 0.6 | 1.0 | 2.7 |

**[Table 4]**

| Measurement results of number of IgA producing cells | | | | |
|---|---|---|---|---|
| | Total number of IgA producing cells (cells/10⁶cells) | | Number of PC-specific IgA antibody producing cells (cells/10⁶cells) | |
| | Control group | Administrati on group | Control group | Administrati on group |
| Measured value | 83 | 126 | 26 | 29 |
| | 100 | 140 | 20 | 37 |
| Average value | 92 | 133 | 23 | 33 |

### Example 5

### Evaluation-3 of IgA secretion promoting action by repeated mixed feed administration to mice

A test feed including a normal feed (MF, Oriental Yeast Co., Ltd.) mixed with a corn poppy (flower) hot water extract at 5% (w/w) was prepared. Twenty 7-week-old mice (BALB/c) were divided into a control group and a test group (ten mice for each group). The total amount of an IgA antibody in the sinus mucus, PC-specific IgA antibody titer, and total amount of an IgA antibody in the feces were measured in the same manner as in Example 4.

The results are as shown in Table 5. The administration group showed a higher value for the total amount of IgA in the sinus mucus. This suggests that the mixed feed administration of the corn poppy extract promoted IgA secretion in the respiratory system.

**[Table 5]**

| Measurement results of IgA level | | | | | | |
|---|---|---|---|---|---|---|
| | Sinus mucus | | | | Feces | |
| | Total amount of IgA (ng/ml) | | PC-specific IgA antibody titer (Reciprocal log2 titer) | | Total amount of IgA (µg/ml) | |
| | Control group | Administration group | Control group | Administration group | Control group | Administration group |
| Measured value for each individual | 424 | 553 | 6 | 7 | 2.0 | 8.3 |
| | 574 | 435 | 6 | 6 | 2.2 | 0.9 |
| | 422 | 254 | 8 | 6 | 2.9 | 3.9 |
| | 277 | 525 | 5 | 6 | 3.7 | 2.8 |
| | 683 | 779 | 6 | 7 | 1.9 | 8.1 |
| | 175 | 672 | 6 | 7 | 4.4 | 1.3 |
| | 272 | 176 | 6 | 6 | 3.3 | 1.4 |
| | 191 | 254 | 8 | 6 | 2.7 | 1.7 |
| | 231 | 289 | 5 | 6 | 2.2 | 2.1 |
| | 303 | 287 | 6 | 7 | 2.1 | 2.2 |
| Average value | 355 | 422 | 6.2 | 6.4 | 2.7 | 3.3 |
| Standard error | 50.5 | 60.9 | 0.31 | 0.15 | 0.25 | 0.82 |

### Example 6

### Evaluation-1 of IgA secretion promoting action by repeated sublingual administration to mice

7-week-old mice (BALB/c) were divided into a control group and an administration group (four mice for each group) and fed with a normal feed (MF, Oriental Yeast Co., Ltd.). From the start of the feeding, a corn poppy (flower) hot water extract was sublingually administered to the administration group daily at a dose of 4 mg/day in a form of an aqueous solution, whereas an equal amount of water was sublingually administered alone to the control group. On week 4, the mice were immunized with 50 µg of PC-KLH by transnasal administration. After that, the transnasal immunization was carried out every 1 week, sinus mucus and lymphocyte cells in the lamina propria mucosae in the nasal cavity were collected 1 week after the third transnasal immunization, and the total amount of an IgA antibody and the total number of IgA antibody producing cells were measured. The total amount of the IgA antibody was measured by a sandwich ELISA method in the same manner as in Example 2, and the number of the IgA antibody producing cells was measured by an enzyme-linked immunospot (ELISPOT) method in the same manner as in Example 4. Since the number of the lymphocyte cells in the lamina propria mucosae in the nasal cavity capable of being harvested from each animal was very small, the number of the IgA antibody producing cells was measured in duplicate by combining the cells from four mice together.

The results are as shown in Table 6 and Table 7. The administration group showed higher values for the total amount of IgA and the total number of the IgA producing cells in the sinus mucus. This suggests that the sublingual administration of the corn poppy extract promoted IgA secretion in the respiratory system, resulting in an increase in the number of the IgA producing cells.

**[Table 6]**

| | Total amount of IgA (ng/ml) | |
|---|---|---|
| | Control group | Administration group |
| Measured value for each individual | 247 | 265 |
| | 262 | 222 |
| | 155 | 165 |
| | 240 | 619 |
| Average | 226 | 318 |

**[Table 7]**

| | Total number of IgA producing cells (cells/10⁶ cells) | |
|---|---|---|
| | Control group | Administration group |
| Each measured value | 500 | 790 |
| | 320 | 900 |
| Average value | 410 | 845 |

### Example 7

### Evaluation-2 of IgA secretion promoting action by repeated sublingual administration to mice

7-week-old mice (BALB/c) were divided into a control group and an administration group (fifteen mice for each group) and fed with a normal feed (MF, Oriental Yeast Co., Ltd.). From the start of the feeding, a corn poppy (flower) hot water extract was sublingually administered to the administration group daily at a dose of 4 mg/day in a form of an aqueous solution, whereas an equal amount of water was sublingually administered alone to the control group. On week 4, the mice were immunized with 50 µg of PC-KLH by transnasal administration. After that, the transnasal immunization was carried out every 1 week, sinus mucus and lymphocyte cells in the lamina propria mucosae in the nasal cavity were collected 1 week after the third transnasal immunization, and the total amount of an IgA antibody, the total number of IgA antibody producing cells, the PC-specific IgA antibody titer, and the number of PC-specific IgA antibody producing cells were measured. Further, the feces were collected, suspended in a phosphate buffer, and centrifuged. The total amount of the IgA antibody in the supernatant was then measured. The total amount of the IgA antibody and the PC-specific IgA antibody titer were each measured by a sandwich ELISA method in the same manner as in Example 2, and the number of the IgA antibody producing cells was measured by an enzyme-linked immunospot (ELISPOT) method in the same manner as in Example 4. Since the number of the lymphocyte cells in the lamina propria mucosae in the nasal cavity capable of being harvested from each animal was very small, the number of the IgA antibody producing cells was measured in duplicate by combining the cells from three mice together.

The results are as shown in Table 8 and Table 9. The administration group showed higher values for all the items than the control group. This suggests that the sublingual administration of the corn poppy flower extract increased the number of the IgA producing cells and the amount of IgA secretion in both the intestinal tract and the respiratory system.

**[Table 8]**

| Measurement results of IgA level | | | | | | |
|---|---|---|---|---|---|---|
| | Sinus mucus | | | | Feces | |
| | Total amount of IgA (ng/ml) | | PC-specific IgA antibody titer (Reciprocal log2 titer) | | Total amount of IgA (µg/ml) | |
| | Control group | Administration group | Control group | Administration group | Control group | Administrati on group |
| Measured value for each individual | 168 | 271 | 6 | 7 | 8.1 | 15.6 |
| | 178 | 308 | 6 | 7 | 11.4 | 12.8 |
| | 207 | 397 | 6 | 8 | 5.1 | 2.8 |
| | 372 | 382 | 5 | 7 | 2.1 | 5.7 |
| | 194 | 415 | 6 | 7 | 1.2 | 10.1 |
| | 288 | 291 | 6 | 6 | 3.0 | 4.0 |
| | 299 | 700 | 7 | 8 | 4.2 | 10.1 |
| | 601 | 588 | 7 | 7 | 1.6 | 13.3 |
| | 245 | 260 | 6 | 6 | 4.1 | 6.9 |
| | 342 | 293 | 8 | 8 | 4.1 | 1.1 |
| | 359 | 215 | 8 | 6 | 7.1 | 3.7 |
| | 381 | 500 | 7 | 8 | 4.4 | 2.5 |
| | 178 | 391 | 7 | 8 | 0.6 | 2.1 |
| | 298 | 370 | 7 | 7 | 4.8 | 1.7 |
| | 357 | 364 | 7 | 7 | 4.5 | 2.6 |
| Average value | 298 | 383 | 6.6 | 7.1 | 4.4 | 6.3 |
| Standard error | 29 | 34 | 0.2 | 0.2 | 0.7 | 1.2 |

**[Table 9]**

| Measurement results of number of IgA producing cells | | | | |
|---|---|---|---|---|
| | Total number of IgA producing cells (cells/10⁶ cells) | | Number of PC-specific IgA antibody producing cells (cells/10⁶cells) | |
| | Control group | Administration group | Control group | Administration group |
| Measured value for each set | 275 | 467 | 83 | 300 |
| | 267 | 433 | 175 | 308 |
| | 321 | 548 | 155 | 222 |
| | 417 | 571 | 166 | 238 |
| | 764 | 921 | 240 | 213 |
| | 455 | 837 | 223 | 201 |
| | 680 | 771 | 359 | 500 |
| | 555 | 802 | 398 | 458 |
| | 516 | 703 | 375 | 450 |
| | 547 | 813 | 419 | 513 |
| Average value | 480 | 686 | 259 | 340 |
| Standard error | 53 | 54 | 38 | 40 |

### Example 8

### Evaluation-1 of IgA secretion promoting action by repeated transnasal administration to mice

7-week-old mice (BALB/c) were divided into a control group and an administration group (six mice for each group) and fed with a normal feed (MF, Oriental Yeast Co., Ltd.). From the start of the feeding, a corn poppy (flower) hot water extract was transnasally administered to the administration group daily at a dose of 2 mg/day in a form of an aqueous solution, whereas an equal amount of water was transnasally administered alone to the control group. On week 4, the mice were immunized with 50 µg of PC-KLH by transnasal administration. After that, the transnasal immunization was carried out every 1 week, sinus mucus and lymphocyte cells in the lamina propria mucosae in the nasal cavity were collected 1 week after the third transnasal immunization, and the total amount of an IgA antibody and the total number of IgA antibody producing cells were measured. The total amount of the IgA antibody was measured by a sandwich ELISA method in the same manner as in Example 2, and the number of the IgA antibody producing cells was measured by an enzyme-linked immunospot (ELISPOT) method in the same manner as in Example 4. Since the number of the lymphocyte cells in the lamina propria mucosae in the nasal cavity capable of being harvested from each animal was very small, the number of the IgA antibody producing cells was measured in duplicate by combining the cells from three mice together.

The results are as shown in Table 10 and Table 11. The administration group showed higher values for the total amount of IgA and the total number of the IgA producing cells in the sinus mucus. This suggests that the transnasal administration of the corn poppy extract promoted IgA secretion in the respiratory system, resulting in an increase in the number of the IgA producing cells.

**[Table 10]**

| | Total amount of IgA (ng/ml) | |
|---|---|---|
| | Control group | Administration group |
| Measured value for each individual | 327 | 402 |
| | 211 | 569 |
| | 229 | 210 |
| | 392 | 293 |
| | 432 | 487 |
| | 497 | 402 |
| Average | 348 | 394 |

**[Table 11]**

| | Total number of IgA producing cells (cells/10⁶ cells) | |
|---|---|---|
| | Control group | Administration group |
| Each measured value | 400 | 462 |
| | 369 | 523 |
| | 400 | 483 |
| | 300 | 442 |
| Average value | 367 | 477 |

### Example 9

### Evaluation-2 of IgA secretion promoting action by repeated transnasal administration to mice

5- to 6-month-old mice (BALB/c) were divided into a control group and an administration group (five mice for each group) and fed with a normal feed (MF, Oriental Yeast Co., Ltd.). From the start of the feeding, a corn poppy (flower) hot water extract was transnasally administered to the administration group daily at a dose of 2 mg/day in a form of an aqueous solution, whereas an equal amount of water was transnasally administered alone to the control group. On week 4, the mice were immunized with 50 µg of PC-KLH by transnasal administration. After that, the transnasal immunization was carried out every 1 week, sinus mucus and lymphocyte cells in the lamina propria mucosae in the nasal cavity were collected 1 week after the third transnasal immunization, and the total amount of an IgA antibody and the total number of IgA antibody producing cells were measured. The total amount of the IgA antibody was measured by a sandwich ELISA method in the same manner as in Example 2, and the number of the IgA antibody producing cells was measured by an enzyme-linked immunospot (ELISPOT) method in the same manner as in Example 4. Since the number of the lymphocyte cells in the lamina propria mucosae in the nasal cavity capable of being harvested from each animal was very small, the number of the IgA antibody producing cells was measured in duplicate by combining the cells from two to three mice together.

The results are as shown in Table 12 and Table 13. The administration group showed higher values for the total amount of IgA and the total number of the IgA producing cells in the sinus mucus. This suggests that the transnasal administration of the corn poppy extract promoted IgA secretion in the respiratory system, resulting in an increase in the number of the IgA producing cells.

**[Table 12]**

| | Total amount of IgA (ng/ml) | |
|---|---|---|
| | Control group | Administration group |
| Measured value for each individual | 220 | 349 |
| | 531 | 1,020 |
| | 688 | 538 |
| | 499 | 618 |
| | 424 | 734 |
| Average | 472 | 652 |

**[Table 13]**

| | Total number of IgA producing cells (cells/10⁶cells) | |
|---|---|---|
| | Control group | Administration group |
| Each measured value | 769 | 1,077 |
| | 692 | 1,000 |
| | 1,091 | 1,227 |
| | 773 | 1,227 |
| Average value | 831 | 1,133 |

In addition, a pill, a powder, an inhalant, a nasal drop, chewing gum, a candy, chocolate, a biscuit, a gummy jelly, a confectionery tablet, an ice cream, a sherbet, and a beverage were prepared by a conventional method through the use of the corn poppy extract prepared in Example 1. The formulations are shown below. It should be noted that the formulations are not intended to limit the scope of the products of the present invention.

### Example 10

A pill was prepared according to the following formulation.

| | |
|---|---|
| D-mannitol | 35.0% |
| Lactose | 27.5 |
| Crystalline cellulose | 12.5 |
| Hydroxypropyl cellulose | 5.0 |
| Corn poppy extract of Example 1 | 20.0 |
| | 100.0% |

### Example 11

A pill was prepared according to the following formulation.

| | |
|---|---|
| Lactose | 54.5% |
| Corn starch | 38.5 |
| Magnesium stearate | 2.0 |
| Corn poppy extract of Example 1 | 5.0 |
| | 100.0% |

### Example 12

A capsule was prepared according to the following formulation.

| | |
|---|---|
| Lactose | 8.0% |
| Magnesium stearate | 2.0 |
| Corn poppy extract of Example 1 | 90.0 |
| | 100.0% |

### Example 13

A powder was prepared according to the following formulation.

| | |
|---|---|
| Lactose | 40.0% |
| Potato starch | 15.0 |
| Corn poppy extract of Example 1 | 45.0 |
| | 100.0% |

### Example 14

An inhalant was prepared according to the following formulation.

| | |
|---|---|
| Ethanol | 5.0% |
| Corn poppy extract of Example 1 | 0.5 |
| L-Menthol | 1.0 |
| Water | 93.5 |
| | 100.0% |

### Example 15

A nasal drop was prepared according to the following formulation.

| | |
|---|---|
| Methyl salicylate | 0.03 g |
| Chlorophenylamine maleate | 0.3 |
| dl-Methylephedrine hydrochloride | 0.3 |
| Polysorbate 80 | 0.2 |
| Benzalkonium chloride | 0.01 |
| Sodium chloride | 0.6 |
| 1 N sodium hydroxide | q.s. |
| Corn poppy extract of Example 1 | 0.01 |
| Water | q.s. |
| | 100.0 ml (pH 6.8) |

### Example 16

Chewing gum was prepared according to the following formulation.

| | |
|---|---|
| Gum base | 20.0% |
| Sugar | 52.0 |
| Glucose | 14.0 |
| Starch syrup | 13.0 |
| Flavoring agent | 0.5 |
| Corn poppy extract of Example 1 | 0.5 |
| | 100.0% |

### Example 17

Chewing gum was prepared according to the following formulation.

| | |
|---|---|
| Gum base | 20.0% |
| Sugar | 53.0 |
| Glucose | 15.0 |
| Starch syrup | 10.5 |
| Flavoring agent | 0.5 |
| Corn poppy extract of Example 1 | 1.0 |
| | 100.0% |

### Example 18

Chewing gum was prepared according to the following formulation.

| | |
|---|---|
| Gum base | 20.0% |
| Sugar | 54.0 |
| Glucose | 14.0 |
| Starch syrup | 9.3 |
| Flavoring agent | 0.7 |
| Corn poppy extract of Example 1 | 2.0 |
| | 100.0% |

### Example 19

A candy was prepared according to the following formulation.

| | |
|---|---|
| Sugar | 45.0% |
| Starch syrup | 40.0 |
| Flavoring agent | 0.5 |
| L-Menthol | 0.3 |
| Corn poppy extract of Example 1 | 0.2 |
| Water | 14.0 |
| | 100.0% |

### Example 20

A candy was prepared according to the following formulation.

| | |
|---|---|
| Sugar | 50.0% |
| Starch syrup | 33.0 |
| Citric acid | 1.0 |
| Flavoring agent | 0.6 |
| Corn poppy extract of Example 1 | 1.0 |
| Water | 14.4 |
| | 100.0% |

### Example 21

Chocolate was prepared according to the following formulation.

| | |
|---|---|
| Cacao bitter | 20.0% |
| Whole milk powder | 20.0 |
| Cacao butter | 17.0 |
| Powder sugar | 41.7 |
| Lecithin | 0.5 |
| Flavoring agent | 0.2 |
| Corn poppy extract of Example 1 | 0.6 |
| | 100.0% |

### Example 22

A biscuit was prepared according to the following formulation.

| | |
|---|---|
| Sugar | 31.0% |
| Wheat | 26.5 |
| Starch powder | 26.5 |
| Butter | 4.0 |
| Egg | 10.5 |
| Baking soda | 0.3 |
| Corn poppy extract of Example 1 | 1.2 |
| | 100.0% |

### Example 23

A gummy jelly was prepared according to the following formulation.

| | |
|---|---|
| Aqueous polydextrose solution | 38.0% |
| Aqueous sorbitol solution | 8.0 |
| Aqueous palatinose solution | 9.0 |
| Aqueous maltose solution | 20.0 |
| Aqueous trehalose solution | 11.0 |
| Gelatin | 10.0 |
| Tartaric acid | 1.0 |
| Corn poppy extract of Example 1 | 3.0 |
| | 100.0% |

### Example 24

A confectionery tablet was prepared according to the following formulation.

| | |
|---|---|
| Sugar | 76.0% |
| Glucose | 20.0 |
| Sucrose fatty acid ester | 0.2 |
| Flavoring agent | 0.1 |
| Corn poppy extract of Example 1 | 0.5 |
| Water | 3.2 |
| | 100.0% |

### Example 25

A confectionery tablet was prepared according to the following formulation.

| | |
|---|---|
| Sugar | 72.0% |
| Lactose | 19.8 |
| Sucrose fatty acid ester | 0.2 |
| Corn poppy extract of Example 1 | 5.0 |
| Water | 3.0 |
| | 100.0% |

### Example 26

A tablet was prepared according to the following formulation.

| | |
|---|---|
| Sugar | 28.8% |
| Sucrose fatty acid ester | 0.2 |
| Corn poppy extract of Example 1 | 70.0 |
| Water | 1.0 |
| | 100.0% |

### Example 27

An ice cream was prepared according to the following formulation.

| | |
|---|---|
| Egg yolk | 11.0% |
| Sugar | 13.5 |
| Cow milk | 37.0 |
| Fresh cream | 37.0 |
| Vanilla beans | 0.5 |
| Corn poppy extract of Example 1 | 1.0 |
| | 100.0% |

### Example 28

A sherbet was prepared according to the following formulation.

| | |
|---|---|
| Orange juice | 20.0% |
| Sugar | 29.5% |
| Corn poppy extract of Example 1 | 0.5 |
| Water | 50.0 |
| | 100.0% |

### Example 29

A beverage was prepared according to the following formulation.

| | |
|---|---|
| Orange juice | 40.0% |
| Isomerized sugar | 15.3 |
| Citric acid | 0.1 |
| Vitamin C | 0.05 |
| Flavoring agent | 0.1 |
| Corn poppy extract of Example 1 | 0.05 |
| Water | 44.4 |
| | 100.0% |

### Example 30

A beverage was prepared according to the following formulation.

| | |
|---|---|
| Grape juice | 70.0% |
| Isomerized sugar | 12.0 |
| Citric acid | 0.1 |
| Vitamin C | 0.05 |
| Flavoring agent | 0.1 |
| Corn poppy extract of Example 1 | 0.1 |
| Water | q.s. |
| | 100.0% |

## Claims

1. An IgA secretion promoter, comprising a corn poppy extract.

2. A pharmaceutical composition, comprising the IgA secretion promoter according to claim 1 at 0.01% to 90% in terms of dry weight.

3. The pharmaceutical composition according to claim 2, wherein the pharmaceutical composition is ingested orally.

4. The pharmaceutical composition according to claim 2, wherein the pharmaceutical composition is ingested by sublingual administration.

5. The pharmaceutical composition according to claim 2, wherein the pharmaceutical composition is ingested by transnasal administration.

6. A food, comprising the IgA secretion promoter according to claim 1.

7. A pharmaceutical composition, comprising the IgA secretion promoter according to claim 1.
